# EUROPEAN PATENT SPECIFICATION

(11) **EP 3 766 545 B1**
(45) Date of publication and mention of the grant of the patent: **16.08.2023**
(21) Application number: 19186785.2
(22) Date of filing: 17.07.2019
(51) Int. Cl.: A61P 43/00, A61K 31/198, A61K 31/7028, A61K 8/44, A61Q 7/00, A61K 8/60, A61K 31/7105, A61K 8/49

(54) **ACTIVE AGENT FOR USE IN HAIR GROWTH REGULATION**
WIRKSTOFF ZUR VERWENDUNG FÜR DIE HAARWUCHSREGULIERUNG
AGENT ACTIF DESTINÉ À ÊTRE UTILISÉ DANS LA RÉGULATION DE LA CROISSANCE DES CHEVEUX

(43) Date of publication of application: 20.01.2021
(73) Proprietor: CUTANEON - Skin & Hair Innovations GmbH, 22339 Hamburg (DE)
(72) Inventor: ROUILLE, Thomas, 48147 Münster (DE); CHERÉT, Jeremy, 33156 Miami, Florida (US); WARD, Chris, High Peak, Derbyshire SK23 7BL (GB); PAUS, Ralf, 22339 Hamburg (DE)
(74) Representative: WSL Patentanwälte Partnerschaft mbB

(56) References cited:
- DE-A1-102008 038 242
- JP-A- H0 543 429
- KR-A- 20130 087 094
- US-A1- 2016 166 500
- Frederick D Malkinson ET AL: "Colchicine Alopecia", Journal of Investigative Dermatology, 1 October 1959 (1959-10-01), pages 371-384, XP055655372, United States DOI: 10.1038/jid.1959.162 Retrieved from the Internet: URL:https://www.sciencedirect.com/science/ article/pii/S0022202X15493364?via%3Dihub
- DATABASE GNPD [Online] MINTEL; 17 March 2016 (2016-03-17), anonymous: "Hair Loss Control Fortifying Conditioner", XP055805136, Database accession no. 3870759
- Baar H.M.J. ET AL: "Dapsone versus topical immunotherapy in alopecia areata", British Journal of Dermatology, vol. 133, no. 2, 1 August 1995 (1995-08-01), pages 270-274, XP055805138, UK ISSN: 0007-0963, DOI: 10.1111/j.1365-2133.1995.tb02628.x Retrieved from the Internet: URL:https://core.ac.uk/download/16111354.p df>

## Description

The present invention is directed to an active agent for use in hair growth regulation, particularly for use in the treatment of hair growth, wherein said active agent modulates the activity of a taste receptor, including but not limited to activation, enhancement, inactivation, blocking or dampening the cellular response of a taste receptor or interfering with the expression of said receptor. Further, the present invention is directed to a composition for use as a cosmetic or medicament in the treatment of hair growth said composition comprising at least one active agent that modulates the activity of a taste receptor, including but not limited to activation, enhancement, inactivation, blocking or dampening the cellular response of a taste receptor or interfering with the expression of said receptor. In addition, the present invention is directed to a non-therapeutic method of hair growth regulation, wherein an effective amount of at least one active agent that modulates the activity of a taste receptor, including but not limited to activation, enhancement, inactivation, blocking or dampening the cellular response of a taste receptor or interfering with the expression of said receptor, is administered to a subject.

Evolutionary studies suggest the common ancestor to the hair follicle was a simple glandular structure with a function in regulating hydration, potentially to allow independence of the organism from aquatic environments. Specialization of these simple glands resulted in the evolution of hair follicles with their associated sebaceous and, in some cases, apocrine sweat glands.

The pilosebaceous unit (PSU), an anatomical compartmentalisation of the hair follicle and surrounding parts, consists of the hair follicle, arrector pili muscle, sebaceous gland and, where present, apocrine sweat gland. The hair follicle is an epithelial-mesenchymal-neuroectodermal interaction unit that is the most densely innervated structure of mammalian skin, and in addition, hair follicle possesses its own immune system. As such, the hair follicle is much more than just a hair-producing organ.

The formation of hair follicles occurs once in the lifetime of healthy mammals, with the number of hair follicles determined in utero. Hair follicle morphogenesis is the generation of the whole hair follicle structure from the epidermis and the mesoderm. During postnatal life the hair follicle undergoes life-long cyclical transformations where it progresses through stages of rapid growth (anagen), regression (catagen) and relative quiescence (telogen) classified by morphological indicators and molecular markers.

There are a number of common and rare diseases associated with the hair follicle cycle, such as non-scarring alopecia, scarring alopecia, chronic and acute hair shedding, hypertrichosis or hirsutism. Further, there are a number of other conditions, where the control of unwanted hair on the human body or the promotion of hair growth on areas of the human body is desired, such as drug-induced hair loss (e.g. chemotherapy), radiation-induced hair loss (e.g. radiotherapy), unwanted hair growth due to drugs such as cyclosporine A or diazoxide.

US 2016166500 discloses a hair tonic composition comprising inter alia abscisic acid, indole butyric acid and lanthanum. Said hair tonic composition shall be used for producing hair growth or for preventing hair fall. KR 20130087094 describes compositions useful for treating alopecia, wherein said compositions contain inter alia tryptophan. JP H0543429 describes a hair tonic cosmetic comprising inter alia γ-aminobutyric acid. Said hair tonic cosmetic shall be used in the prevention of alopecia. DE 10 2008 038242 A1 discloses the use of an alcoholic preparation comprising inter alia camphor in the treatment of alopecia areata. The company Universe Garden Angels offers a hair-loss control conditioner comprising inter alia quinine. Baar, H.M.J. et al. discuss in "Dapsone versus topical immunotherapy in alopecia areata" (British Journal of Dermatology, vol. 133, no. 2, p. 270-274) the potential use of dapsone in the treatment of alopecia areata.

There is a continuous need for active agents for actively controlling hair growth in subjects without undesired side effects. The inventors of the present invention found that a taste receptor TAS2R4 was expressed in the hair follicle epithelium (outer root sheath, inner root sheath, and hair matrix) and in the dermal papilla, suggesting it is involved in regulation of hair growth.

Further, the inventors of the present application have found that inactivation of TAS2R4 in anagen hair follicles using RNAi or a TAS2R4 inverse agonist, such as Nα,Nα-bis(carboxymethyl)-l-lysine (BCML) results in hair growth promotion, delayed catagen promotion, increase in hair follicle proliferation and pigmentation, associated with upregulation of the expression of pro-anagenic growth factor IGF1 and downregulation of pro-catagenic growth factor TGFβ2. *Vice versa,* activation of TAS2R4 using agonists, such as Rebaudioside A, Rubusoside and Stevioside, results in hair growth inhibition, accelerated anagen-catagen transition, associated with the reduced hair follicle cell proliferation and pigmentation.

Concluding, the inventors of the present application have found that the administration of an active agent that activates, enhances, inactivates, blocks or dampens the cellular response of a receptor or interferes with the expression of the taste receptor TAS2R4 is effective in hair growth regulation.

A "taste receptor" originally describes a type of receptor that facilitates the sensation of taste in the oral cavity. Taste receptors are divided into two families, type 1 (sweet, TAS1 R2 - TAS1 R3), and type 2 (bitter, TAS2R1 - TAS2R50, and TAS2R60). Combinations of these receptors in dimers or other complexes contribute to different perceptions of taste. The presence of TAS2R4 is not previously known in hair follicle cells.

The mRNA expression of some but not all TAS2Rs, including (TAS2R3, TAS2R4, TAS2R5, TAS2R8, TAS2R9 TAS2R14, TAS2R30, TAS2R42, and TAS2R60) was detected in the RNA collected from full-thickness skin biopsies (Shaw et al., 2018). Expression of bitter taste receptors TAS2R1 and TAS2R38 has been reported in human epidermal keratinocytes (Wölfle et al., 2015).

A number of bitter taste receptor have been reported to be expressed in extraoral/nasal tissues, such as but not limited to the reproductive organs, the upper respiratory tract, the gastrointestinal tract, the brain and the immune system (Behrens et Meyerhof, 2018; Luddi et al., 2019; Patel et al., 2018; Tran et al., 2018).

"TAS2R4" is the official gene symbol for "Taste receptor type 2 member 4" and identifies the protein that is encoded by the TAS2R4 gene in humans (NCBI Gene ID: 50832; HGNC: 14911; NCBI mRNA sequence: NM_016944.1; NCBI protein sequence: NP_058640). The protein is a 7-transmembrane receptor protein, member of the G protein-coupled receptor superfamily and functions as a bitter taste receptor. TAS2R4 RNA expression levels are highest in the skin, reproductive organs, brain and endocrine tissues.

According to one alternative of the present invention an active agent that activates, enhances, inactivates, blocks or dampens the cellular response of the taste receptor TAS2R4 is used in the treatment or regulation of hair growth, wherein said active agent is an agonist or antagonist of the taste receptor TAS2R4.

An active agent that activates, enhances, inactivates, blocks or dampens the cellular response of a receptor is either an agonist or antagonist/inverse agonist of the receptor, wherein an "agonist" is a substance that binds to a receptor and activates or enhances the receptor to produce the cellular response. An "antagonist" is a substance that binds to a receptor and blocks or dampens the cellular response to an agonist rather than activating or enhancing it like an agonist. An "inverse agonist" is a substance that binds to a receptor and inactivates the cellular response to an agonist rather than activating or enhancing it like an agonist.

Accordingly an inventive agonist of the taste receptor TAS2R4 activates or enhances the TAS2R4 receptor to produce the cellular response, whereas the inventive antagonist of the taste receptor TAS2R4 blocks or dampens the TAS2R4 receptor response to endogenous agonists, and the inventive inverse agonist of the taste receptor TAS2R4 inactivates the TAS2R4 receptor response to endogenous agonists, to produce the cellular response.

In those embodiments, where the active agent is an agonist of the taste receptor TAS2R4, said active agent is used in the treatment of unwanted hair growth. In those embodiments, where the active agent is an antagonists/inverse agonists of the taste receptor TAS2R4, said active agent is used in the treatment of unwanted hair loss.

In specific embodiments of the invention the taste receptor agonist/antagonist/inverse agonist used is a TAS2R4 agonist selected from the group consisting of amarogentin, arborescin, artemorin, azathioprine, chlorpheniramine, denatonium benzoate, diphenidol, dulcoside A, (-)-epicatechin, leu-leu-leu, leu-trp, parthenolide, propylthiouracil, phe-trp, quassin, rebaudioside A, rebaudioside B, rebaudioside C, rubusoside, steviolbioside, stevioside, sucralose, taurocholic acid, trp-leu, trp-phe, trp-pro, trp-trp, trp-trp-trp, xanthoxin, yohimbine, and combinations thereof. Alternatively, the TAS2R4 agonist is an aptamer binding to the TAS2R4 receptor and activating or enhancing the receptor to produce the cellular response.

The term "aptamer" as used herein refers to DNA, RNA or XNA oligonucleotide or peptide molecules that bind to a specific target molecule, such as receptor molecules.

In other embodiments of the invention the taste receptor agonist/antagonist/inverse agonist used is a TAS2R4 antagonist/inverse agonist selected from the group consisting of carboxymethyllysine (CML), Nα,Nα-bis(carboxymethyl)-L-lysine hydrate (BCML), glyoxal-derived lysine dimer (GOLD), 4-(2,2,3-trimethylcyclopentyl) butanoic acid (GIV3727), and combinations thereof. Alternatively, the TAS2R4 antagonist/inverse agonist is an aptamer binding to the TAS2R4 receptor and inactivating, blocking or dampening the receptor to produce the cellular response.

According to an alternative of the present invention, an active agent that interferes with the expression of the taste receptor TAS2R4 is used in hair growth regulation. An active agent that interferes with the expression of the taste receptor TAS2R4 is either miRNA, siRNA or a ribozyme targeted to the TAS2R4 gene or targeted to the mRNA corresponding to the TAS2R4 gene.

The term "miRNA" refers to microRNA, i.e. small non-coding RNA molecules containing 21 to 23 nucleotides and functioning in RNA knock-down and post-transcriptional regulation of gene expression. miRNAs function via base-pairing with complementary sequences within mRNA molecules. As a result, these mRNA molecules are knocked-down, by cleavage, destabilization and/or less efficient translation of the mRNA.

The term "siRNA" refers to small interfering RNA, i.e. small non-coding RNA molecules, 20 to 25 base pairs in length. siRNA interferes with the expression of specific genes with complementary nucleotide sequences by degrading mRNA after transcription, thereby preventing translation.

According to the present invention a gene is "targeted" by a miRNA, siRNA or a ribozyme when the miRNA, siRNA or a ribozyme molecule selectively decreases or inhibits the expression of TAS2R4. The phrase "selectively decrease or inhibit" as used herein refers to miRNA, siRNA or a ribozyme that affects the expression of TAS2R4.

In specific embodiments of the invention miRNA or siRNA interfere with the gene expression of the taste receptors TAS1 R3 or TAS2R4 by hybridizing under stringent conditions to the gene transcript, i.e. the TAS1 R1 or TAS2R4 mRNA, wherein hybridizing "under stringent conditions" means annealing to the target mRNA region, under standard conditions, e.g., high temperature (e.g. < 60 °C for 2 hours) and/or low salt content (e.g. 0.1xSSC) which tend to disfavor hybridization.

According to the present invention one of the above-mentioned active agents is used in the treatment of hair growth. In specific embodiments of the invention the treatment is effected locally, i.e. in, on or at the skin area to be treated. In some embodiments of the invention said treatment is effected topically, wherein the term "topical" refers to a medication that is applied to a particular place on the skin and/or its appendages, such as hair. In specific embodiments the topical application is epicutaneous, meaning that the agonist or antagonist is applied directly to the skin. In other embodiments of the invention the treatment is effected transdermally, wherein the term "transdermal" refers to a medication that is applied across the Stratum corneum into the deeper skin layers.

The term "treatment" as used herein refers to any action resulting in the change of a physical condition. Particularly, the "treatment of hair growth" refers to any change of an initial hair growth condition, such as unwanted presence of hair, unwanted lack of hair, unwanted slow/fast hair growth, chemotherapy-related hair loss, drug-related hair growth and radiation-related hair loss. In other words, the present invention is directed to any kind of hair growth regulation.

In specific embodiments of the invention the above-mentioned active agent is used as a cosmetic in the treatment of hair growth. Particularly, the use as a cosmetic occurs non-therapeutically, but in order to achieve a change of an initial hair growth condition, such as unwanted presence of hair, unwanted lack of hair, unwanted slow/fast hair growth, wherein said initial condition is not caused by a disease or disorder.

In those embodiments, where the above mentioned active agent is used as a cosmetic the active agent used should be cosmetically acceptable, wherein "cosmetically acceptable" means that the active agent should not be toxic or harmful or have any other detrimental side effects upon application on hair and/or skin.

In other specific embodiments of the invention the above mentioned active agent is used as a medicament in the topical treatment of hair growth disorder, wherein the term "disorder" refers to any functional abnormality or disturbance of the normal healthy condition, and the term "medicament" refers to a substance useful in curing, treating or preventing a condition of disorder.

In some embodiments the above mentioned active agent is used as a medicament in the topical treatment of a hair growth disorder being selected from the group consisting of nonscarring (non cicatricial) alopecia, such as alopecia areata, telogen effluvium, androgenetic alopecia and anagen effluvium, scarring (cicatricial) alopecia, such as cutaneous lichen planopilaris, discoid lupus erythematosus, dissecting cellulitis and folliculitis decalvans, hypertrichiosis and hirsutism.

In those embodiments, where the above mentioned active agent is used as a medicament the active agent used should be pharmaceutically acceptable, wherein "pharmaceutically acceptable" means that the active agent should not be toxic or harmful or have any other detrimental side effects upon application on hair and/or skin.

In some embodiments at least one of the inventive active is used as an ingredient of a composition for use as a cosmetic or medicament in the topical treatment of hair growth said composition further comprising at least one auxiliary agent selected from the group consisting of carriers, recipients, adjuvants, diluents, and disintegrants.

In specific embodiments of such compositions the auxiliary agent is selected from the group consisting of liposomes, nanoparticles, carboxymethyl cellulose, hydroxyethyl cellulose, mineral oil, petrolatum, glycerin, polysorbat 80, hydroxyethyl starch, dextran, and polyethylene glycol.

In the inventive compositions the concentration of the active agent usually is in the range of from 10 to 10.000 µM. In some embodiments the lower limit for the concentration of the active agent is 30 µM or even 100 µM. In some embodiments the upper limit is 3.000 µM or 1.000 µm. This results in preferred ranges of e.g. from 30 to 10.000 µM, from 30 to 3.000 µM, from 10 to 3.000 µM, 100 to 3.000 µM and the like.

In specific embodiments such compositions further comprise at least one other active agent being effective in the treatment of hair growth.

In such embodiments the other active agent may be selected from hair growth inhibitors, such as inhibitors of omithine decarboxylase (including Difluoromethylornithine (DFMO)), antiandrogen compounds, inhibitors of 5-α-reductase, inhibitors of androgen receptor, inhibitors of S-adenosyl methionine decarboxylase, inhibitors of γ-glutamyl transpeptidase, inhibitors of adenylosuccinate synthetase, inhibitors of aspartate transcarbamylase, inhibitors of transglutaminase, inhibitors of L-asparagine synthetase, pantothenic acid and its analogues, sulfhydryl reactive compounds, inhibitors of lipoxygenase, inhibitors of cyclooxygenase, inhibitors of nitric oxide synthetase, inhibitors of ornithine amino transferase, inhibitors of cysteine synthesis pathway enzymes, inhibitors of protein kinase C, catechin compounds, green tea polyphenols, non-steroidal angiogenesis suppressors, inhibitors of arginase, inhibitors of the metabolic pathway for the conversion of glucose to acetyl-CoA, compounds that inhibit the formation of glycoprotein, proteoglycans, and glycosaminoglycans, inhibitors of matrix metalloproteinase, inhibitors of the cholesterol synthesis pathway, inhibitors of DNA topoisomerase, inhibitors of aminoacyl-tRNA synthetase, inhibitors of the hypusine biosynthesis pathway, compounds that activate androgen conjugation, inhibitors of alkaline phosphatase, inhibitors of protein tyrosine kinase, and compounds that increase cellular ceramide levels. Specific examples include cyproterone acetate, progesterone, acivicin, anthglutin, L-alanosine, guanidino-succinic acid, ethacrynic acid, D-pantothenic acid, pantoyl alcohol, gabaculin, canaline, isonicotinic acid, verapamil, phentolamine, pentosan polysulfate, nafoxidine, tripelennamine, octapine, phloretin, argaric acid, simvastatin, atorvastatin, lovastatin, fluvastatin, mevastatin, N^{G}-methyl-L-arginine, NG-nitro-L-arginine, benzoyl-L-argininamide, L-argininamide, quercetin, apigenin, nordihydroguaratic acid (NDGA), ketoprofen, naproxen, tolmetin, diclofenac, diflunisal, sulindac, thiosalicylic acid, cysteamine, diethyldithiocarbamic acid, D-penicillamine, N-acetyl-L-cysteine, bathocuproine, enalapril, tamoxifen, cimetidine, mycophenolic acid, tetracycline, doxycycline, minocycline, thioridazine, trifluoperizine, 1-(5-isoquinolinylsulfonyl)-2-methylpiperazine, glycyrrhetinic acid, epigallocatechin gallate, epicatechin gallate, epigallocatechin, epicatechin, fusidic acid, and nitroso-acetyl-penicillamine.

In yet other embodiments the other active agent may be selected from preventatives of chemotherapy- or radiation-induced alopecia or hair loss, such as 4-((cyanoimino((1,2,2-trimethylpropyl)amino)methyl)amino)benzonitrile, epidermal growth factor, fibroblast growth factors, including but not limited to keratinocyte growth factor (FGF7), prostaglandins, cyclin dependent kinases, p53 inhibitors, capase-3 inhibitors, N-acyl cysteine, parathyroid hormone antagonist, alpha-tocopherol, cyclosporine, angiotensin receptor blockers, and minoxidil.

Generally, the inventive composition may be used in any formulation suitable for treatment of hair growth. In specific embodiments of the invention the composition is formulated in the form of an ointment, a lotion, a cream, a shampoo, a gel, a spray, a plaster or a sustained release plaster. In other specific embodiments of the invention the composition is formulated in the form of a solution. Said solution may be applied by means of a microneedle device or prior to sonication, electrical stimulation, etc.

As mentioned above, the inventive use of the above mentioned active agent may also occur non-therapeutically, wherein non-therapeutically refers to a treatment not being directed to curing, treating or preventing a condition of disorder (see above).

Therefore, the present invention is further directed to a non-therapeutic method of hair growth regulation, wherein an effective amount of at least one of the above mentioned active agent is administered to a subject.

The non-therapeutic method also encloses embodiments, where the above mentioned active agent is administered to the subject to be treated simultaneously, sequentially or separately together with at least one other active agent useful in the treatment of hair growth (see above).

The following examples show some of the features of specific embodiments of the present invention. However, the skilled reader will understand that those embodiments are just exemplary but do not restrict the inventive idea to exactly the features or the combination of features of the embodiments of the examples.

In the description of the examples it is referred to the following figures, wherein
- Figure 1: shows the result of an immunofluorescence analysis of TAS2R4 expression in human anagen hair follicle (cf. Example 1).
- Figure 2: shows effect of TAS2R4 knockdown on hair follicle biology, siRNA-targeting TAS2R4 (cf. Example 2) (Mean+/- SEM, n=5 HFs/group, GraphPad Prism 6 (GraphPad, San Diego, CA), ***p<0,001, Student's unpaired t-test).
- Figure 3: shows the data of the treatment of human hair follicles with 120µM Nα,Nα-bis(carboxymethyl)-I-lysine hydrate (BCML) (cf. Example 3) (Mean+/- SEM, n=4-7 HFs/group, GraphPad Prism 6).
- Figure 4: shows the data of the treatment of human hair follicles with 200µM L-Tryptophan (cf. Example 4) (Mean+/- SEM, n=6 HFs/group, GraphPad Prism 6).
- Figure 5: shows the data of the treatment of human hair follicles with 200µM Rebaudioside A (cf. Example 5) (Mean+/- SEM, n=4-7 HFs/group from 2 donors, GraphPad Prism 6).
- Figure 6: shows the data of the treatment of human hair follicles with 50µM Rubusoside (cf. Example 6) (Mean+/- SEM, n=6-7 HFs/group, GraphPad Prism 6).
- Figure 7: shows the data of the treatment of human hair follicles with 200µM Stevioside (cf. Example 7) (Mean+/- SEM, n=5-8 HFs/group, GraphPad Prism 6).

### Examples

### 1. Immunofluorescence analysis of TAS2R4 expression in human hair follicles

Cryosection without fixation stored at -80°C was dried for 10 min at room temperature (RT) and then fixed in acetone for 20 min at -20°C. Slides were then washed in Tris-buffered saline (TBS) for 5 min at RT (repeat x2). The sample sections were encircled with a wax marker pen and the slides placed in a humidified chamber. Sample sections were pretreated with 10% normal goat serum diluted in TBS for 20 min at RT. 1:100 dilution of rabbit polyclonal anti-TAS2R4 (ThermoFisher Cat No.: OSR00153W) in green antibody diluent was added to sample sections and incubated overnight (ON) in a humidified chamber at 4°C. Slides were washed in TBS for 5 min at RT (repeat x2). 1 :1000 dilution of goat antirabbit-A488 Ab (ThermoFisher Cat No.: A1 1070) in green antibody diluent was added to sample sections and incubated at RT for 45 min. Slides were washed in TBS for 5 min at RT (repeat x2). Sample sections were incubated with DAPI for 5 min at RT. Slides were washed in TBS for 5 min at RT (repeat x2) and mounted with coverslip using Southernbiotech Fluoromount.

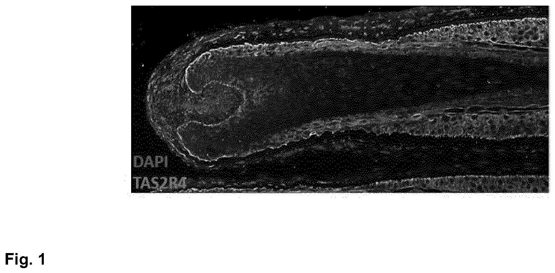

Figure 1 shows the result of the immunofluorescence analysis of TAS2R4 expression in human anagen hair follicle according to the above protocol.

TAS2R4 expression was detected in the outer root sheath, hair matrix, dermal papilla, and connective tissue sheath.

### 2. Inactivation of TAS2R4 by RNAi knockdown

For analysing the effect of inactivating TAS2R4 by RNAi knock-down, hair follicles were cultured in William's E Medium (WEM) supplemented with 2mM L-glutamine, 10ng/mL hydrocortisone, 10µg/mL insulin and 1% penicillin/streptomycin mix (WCM medium), and treated either with self-delivering scrambled oligos (Accell Non-targeting Control siRNA; Horizon Discovery Ltd, cat. D-001910-10-05) or with self-delivering siTAS2R4 (Accel si-TAS2R4; Horizon Discovery Ltd, cat. E-013102-00-0005) according to the following procedure:
Day 0: Isolation of anagen hair follicles
Day 1: Change WCM media with self-delivery siRNA corresponding to experimental groups (Control group: 1µM scrambled oligos, experimental group: 1µM siTAS2R4)
Day 2: Rest
Day 3: Rest
Day 4: Collect 3 hair follicles/groups for qPCR (72h after siRNA delivery)
Day 5: Collect 5 hair follicles/groups for immunofluorescence (96h after siRNA delivery)

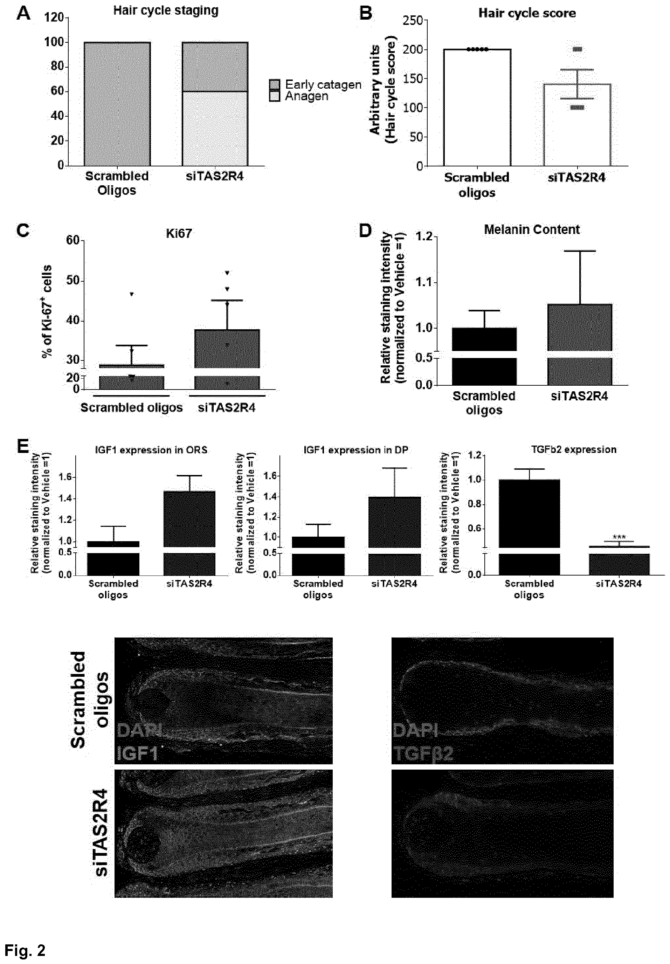

Figure 2 shows the results of siRNA targeting TAS2R4 as mentioned above.

Inactivation of TAS2R4 by RNAi knock-down resulted in the anagen phase prolongation in the hair follicles ex vivo as shown with hair cycle staging and hair cycle score (HCS) (Figure 2A-B). HCS consists of assigning an arbitrary unit for each stage of the hair cycle (Anagen VI = 100; Early catagen = 200; Mid-catagen = 300; and Late catagen = 400). After having classified each HF according to its hair cycle stage, the mean HCS was calculated. The closer the mean is to 100, the higher is the number of anagen VI HFs in a given group. The HCS provides a global readout parameter that looks at all HFs in a given experimental group and synthesizes them into a single number, which reflects how close the majority of HFs is to either anagen VI or catagen and also permits statistical analysis that it is not possible with hair cycle staging. Therefore, hair cycle staging and the HCS are independent readout parameters that complement each other.

TAS2R4 inactivation by RNAi knock-down was also associated with the increased number of proliferative Ki-67+ cells and the increased melanin content (Figure 2C-D).

In addition, the expression of a pro-anagenic growth factor IGF1 and pro-catagenic growth factor TGFβ2 were increased and decreased respectively in response to the TAS2R4 inactivation by RNAi knock-down. (Figure 2E).

### 3. Inactivation of TAS2R4 with Nα,Nα-bis(carboxymethyl)-l-lysine (BCML)

For analysing the effect of inactivating TAS2R4, by the inverse agonist Nα,Nα-bis(carboxymethyl)-l-lysine (BCML), hair follicles were cultured in WEM medium supplemented with 2mM L-glutamine, 10ng/mL hydrocortisone, 10µg/mL insulin and 1% penicillin/streptomycin mix (WCM medium), and treated either with 120µM BCML (Sigma-Aldrich, cat. 14580) diluted in WCM or vehicle (WCM medium) according to the following procedure:
Day 0: Isolation of anagen hair follicles
Day 1: Change media, application of the substance corresponding to experimental groups (control group: WCM, experimental group: 120µM BCML)
Day 2: Rest
Day 3: Change media
Day 4: Rest
Day 5: Change media
Day 6: Rest
Day 7: Collection and freezing of hair follicles for analyses

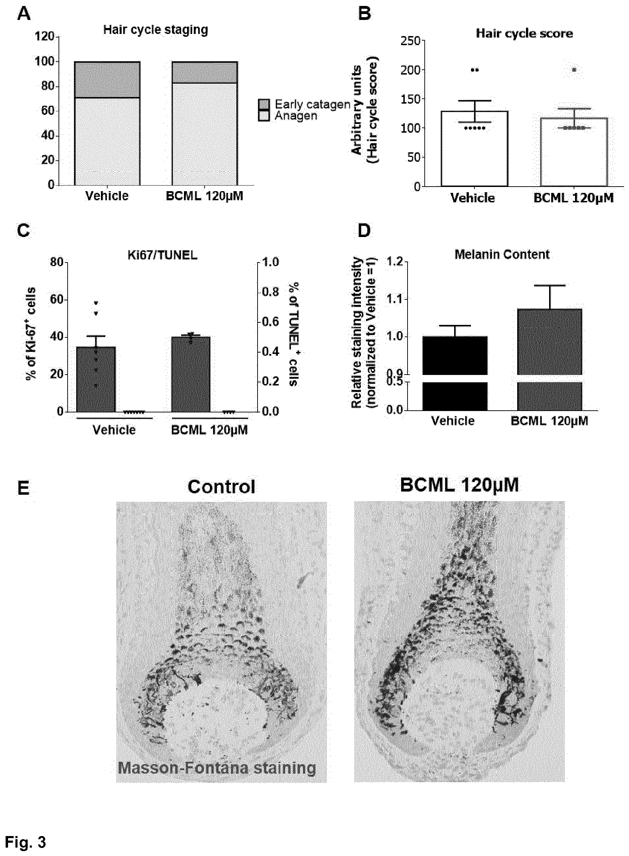

Figure 3 shows the results of human hair follicles treated with 120µM BCML.

Treatment of anagen hair follicles with the BCML increases the proportion of hair follicles in anagen stage (Figure 3A-B).

Further, treatment of human hair follicles with BCML increases hair matrix keratinocyte proliferation while does not affect apoptosis (Figure 3C).

Finally, treatment of hair follicles with BCML increases pigmentation (Figure 3D-E).

### 4. Activation of TAS2R4 with L-Tryptophan

For analysing the effect of activating TAS2R4 by the agonist L-Tryptophan, hair follicles were cultured in WEM medium supplemented with 2mM L-glutamine, 10ng/mL hydrocortisone, 10µg/mL insulin and 1% penicillin/streptomycin mix (WCM medium), and treated either with 200µM L-Tryptophan (Sigma-Aldrich, cat. T8941) diluted in water or vehicle (0,4% water in WCM medium) according to the following procedure:
Day 0: Isolation of anagen hair follicles
Day 1: Change media, application of the substance corresponding to experimental groups (control group: 0,4% water, experimental group: 200µM L-Tryptophan)
Day 2: Rest
Day 3: Change media
Day 4: Rest
Day 5: Change media
Day 6: Rest
Day 7: Collection and freezing of hair follicles for analyses

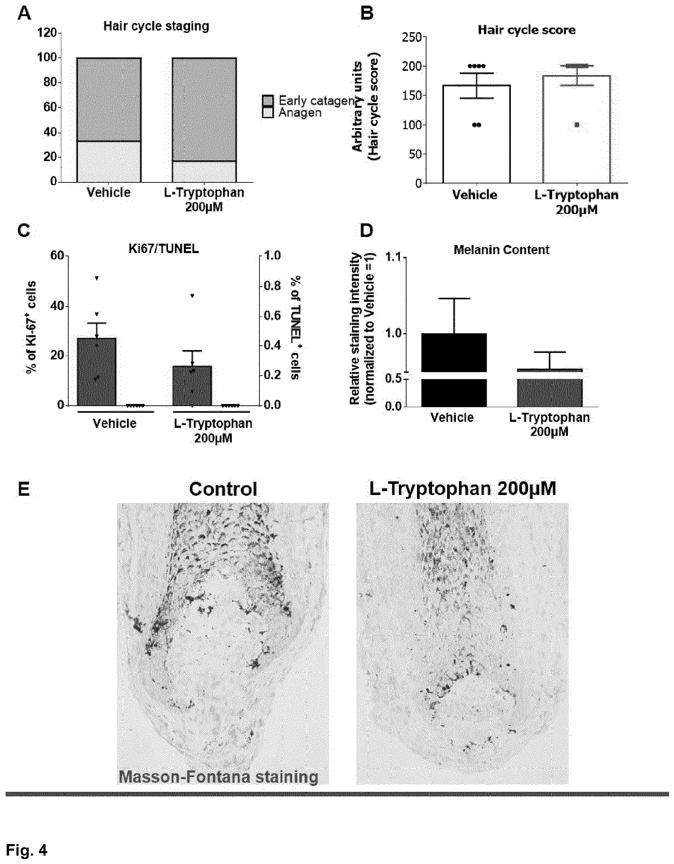

Figure 4 shows the results of human hair follicles treated with 200µM L-Tryptophan.

Activation of TAS2R4 by treatment of anagen hair follicles with the L-Tryptophan increases the proportion of hair follicles in catagen stage (Figure 4A-B).

Further, treatment of human hair follicles with L-Tryptophan decreases hair matrix keratinocyte proliferation while does not affect apoptosis (Figure 4C).

In addition, L-Tryptophan treatment causes hypopigmentation, which is an indication of catagen activation (Figure 4D-E).

### 5. Activation of TAS2R4 with Rebaudioside A

For analysing the effect of activating TAS2R4 by the agonist Rebaudioside A, human anagen hair follicles from 2 donors were cultured in WEM medium supplemented with 2mM L-glutamine, 10ng/mL hydrocortisone, 10µg/mL insulin and 1% penicillin/streptomycin mix (WCM medium), and treated either with 200µM Rebaudioside A (Sigma-Aldrich, cat. 01432) diluted in DMSO or vehicle (0,5% DMSO in WCM medium) according to the following procedure:
Day 0: Isolation of anagen hair follicles
Day 1: Change WCM media, application of the substance corresponding to experimental groups (control group: 0,5% DMSO, experimental group: 200µM Rebaudioside A)
Day 2: Rest
Day 3: Change media
Day 4: Rest
Day 5: Change media
Day 6: Collection and freezing of hair follicles for analyses

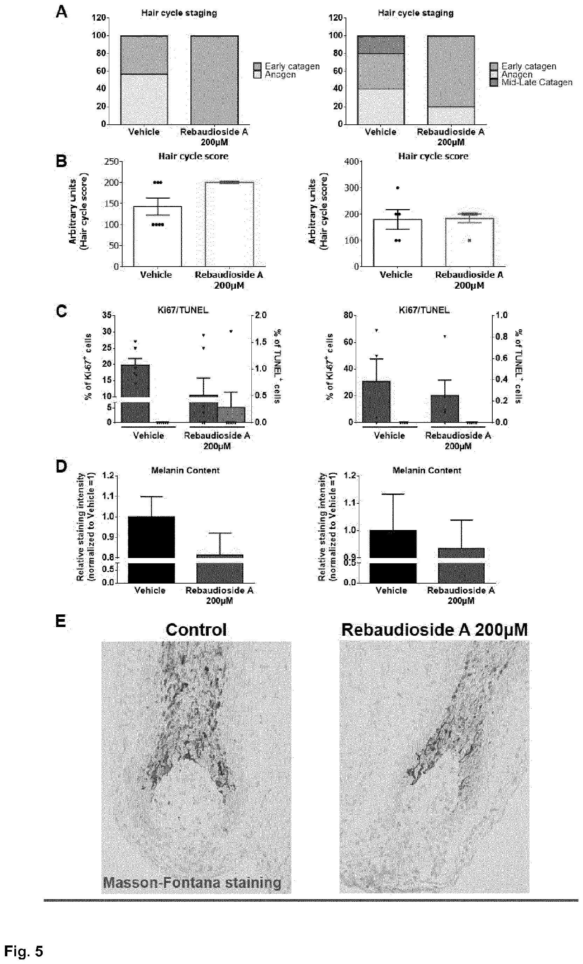

Figure 5 shows the results of human hair follicles treated with 200µM Rebaudioside A.

From said figures it can be identified that activation of TAS2R4 by treatment of hair follicles with the Rebaudioside A increases the proportion of hair follicles in catagen stage (figure 5A-B).

Further, treatment of human hair follicles with Rebaudioside A decreases hair matrix keratinocyte proliferation, and increases apoptosis (figure 5C).

Finally, Rebaudioside A treatment induces hypopigmentation, which is a sign of catagen induction (figure 5D-E).

### 6. Activation of TAS2R4 with Rubusoside

For analysing the effect of activating TAS2R4 by the agonist Rubusoside, hair follicles were cultured in WEM medium supplemented with 2mM L-glutamine, 10ng/mL hydrocortisone, 10µg/mL insulin and 1% penicillin/streptomycin mix (WCM medium), and treated either with 50µM Rubusoside (Sigma-Aldrich, cat. 62933) diluted in DMSO or vehicle (0,05% DMSO in WCM medium) according to the following procedure:
Day 0: Isolation of anagen hair follicles
Day 1: Change media, application of the substance corresponding to experimental groups (control group: 0,05% DMSO, experimental group: 50µM Rubusoside)
Day 2: Rest
Day 3: Change media
Day 4: Rest
Day 5: Change media
Day 6: Collection and freezing of hair follicles for analyses

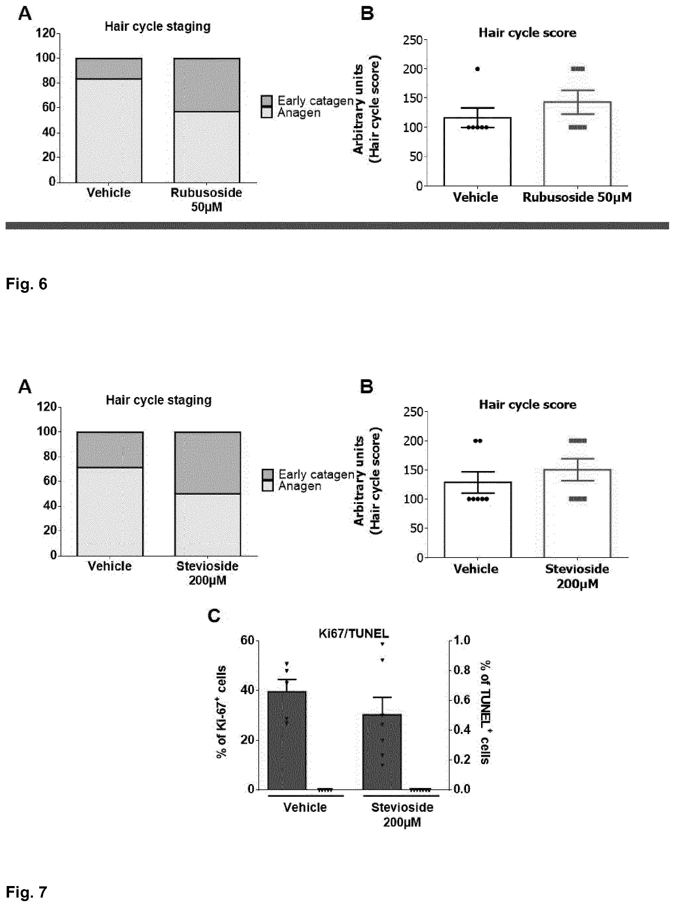

Figure 6 shows the results of human hair follicles treated with 50µM Rubusoside.

From said figures it can be identified that activation of TAS2R4 by treatment of hair follicles with the Rubusoside increases the proportion of human hair follicles in catagen stage (figure 6A-B).

### 7. Activation of TAS2R4 with Stevioside

To determine the effect of activating TAS2R4 by the agonist Stevioside, anagen hair follicles were cultured in WEM medium supplemented with 2mM L-glutamine, 10ng/mL hydrocortisone, 10µg/mL insulin and 1% penicillin/streptomycin mix (WCM medium), and treated either with 200µM Stevioside (Sigma-Aldrich, cat. 50956) diluted in DMSO or vehicle (0,2% DMSO in WCM medium) according to the following procedure:
Day 0: Isolation of anagen hair follicles
Day 1: Change media, application of the substance corresponding to experimental groups (control group: 0,2% DMSO, experimental group: 200µM Stevioside)
Day 2: Rest
Day 3: Change media
Day 4: Rest
Day 5: Change media
Day 6: Rest
Day 7: Collection and freezing of hair follicles for analyses

Figure 7 shows the results of human hair follicles treated with 200µM Stevioside.

From said figures it can be identified that activation of TAS2R4 by treatment of anagen hair follicles with the Stevioside increases the proportion of hair follicles in catagen stage (Figure 7A-B).

Further, treatment of human hair follicles with Stevioside decreases hair matrix keratinocyte proliferation but does not affect apoptosis (Figure 7C).

## Claims

1. Active agent for use in the treatment of a hair growth disorder, wherein said active agent activates, enhances, inactivates, blocks or dampens the cellular response of the taste receptor TAS2R4 or interferes with the expression of said receptor, and wherein the active agent is selected from
a) any one of the TAS2R4 activating agonists amarogentin, arborescin, artemorin, azathioprine, chlorpheniramine, denatonium benzoate, diphenidol, dulcoside A, ()-epicatechin, leu-leu-leu, leu-trp, parthenolide, phe-trp, propylthiouracil, quassin, rebaudioside A, rebaudioside B, rebaudioside C, rubusoside, steviolbioside, stevioside, sucralose, taurocholic acid, trp-leu, trp-phe, trp-pro, trp-trp, trp-trp-trp, xanthoxin, yohimbine, or a combination thereof, or an aptamer binding to the TAS2R4 receptor and activating or enhancing the receptor to produce a cellular response, or
b) any one of the TAS2R4 inactivating antagonists/inverse agonists carboxymethyllysine (CML), Nα,Nα-bis(carboxymethyl)-l-lysine (BCML), glyoxal-derived lysine dimer (GOLD) and 4-(2,2,3-trimethylcyclopentyl) butanoic acid (GIV3727) or a combination thereof, or an aptamer binding to the TAS2R4 receptor and inactivating, blocking or dampening the receptor to produce a cellular response or
c) miRNA, siRNA or a ribozyme targeted to TAS2R4.

2. Active agent for use in the treatment of hair growth according to claim 1, wherein the active agent is an agonist of the taste receptor TAS2R4 for use in the treatment of unwanted hair growth.

3. Active agent for use in the treatment of hair growth according to claim 1, wherein the active agent is an antagonists/inverse agonists of the taste receptor TAS2R4 for use in the treatment of unwanted hair loss.

4. Active agent for use in the treatment of hair growth according to one of the claims 1 to 3, wherein the hair growth disorder to be treated is effluvium, nonscarring alopecia, scarring alopecia, hypertrichosis or hirsutism.

5. Composition for use in the treatment of hair growth said composition comprising at least one active agent according to one of the claims 1 to 3 and at least one auxiliary agent selected from the group consisting of carriers, recipients, adjuvants, diluents, and disintegrants.

6. Composition for use in the treatment of hair growth according to claim 5, wherein the auxiliary agent is selected from the group consisting of liposomes, nanoparticles, carboxymethyl cellulose, hydroxyethyl cellulose, mineral oil, petrolatum, glycerin, polysorbat 80, hydroxyethyl starch, dextran, and polyethylene glycol.

7. Composition for use in the treatment of hair growth according to claim 5 or 6, further comprising at least one other active agent being effective in the treatment of hair growth.

8. Composition for use in the treatment of hair growth according to one of the claims 5 to 7, wherein the composition is formulated in the form of an ointment, a lotion, a cream, a shampoo, a gel, a solution, a spray, a plaster or a sustained release plaster.

9. Non-therapeutic method of hair growth regulation, wherein an effective amount of at least one active agent that activates, enhances, inactivates, blocks or dampens the cellular response of the taste receptor TAS2R4 or interferes with the expression of said receptor is administered to a subject wherein the active agent is selected from
a) any one of the TAS2R4 activating agonists, amarogentin, arborescin, artemorin, azathioprine, chlorpheniramine, denatonium benzoate, diphenidol, dulcoside A, leu-leu-leu, leu-trp, parthenolide, phe-trp, propylthiouracil, quassin, Rebaudioside A, Rebaudioside B, Rebaudioside C, Rubusoside, steviolbioside, Stevioside, sucralose, taurocholic acid, trp-leu, trp-phe, trp-pro, trp-trp, trp-trp-trp, xanthoxin, yohimbine, and (-)epicatechin or a combination thereof, or an aptamer binding to the TAS2R4 receptor and activating or enhancing the receptor to produce a cellular response, or
b) the TAS2R4 inactivating antagonists/inverse agonists carboxymethyllysine (CML), Nα,Nα-bis(carboxymethyl)-l-lysine (BCML), glyoxal-derived lysine dimer (GOLD) and 4-(2,2,3-trimethylcyclopentyl) butanoic acid (GIV3727), or an aptamer binding to the TAS2R4 receptor and inactivating, blocking or dampening the receptor to produce a cellular response or
c) miRNA, siRNA or a ribozyme targeted to the TAS2R4 gene or targeted to the mRNA corresponding to the TAS2R4 gene.

10. Non-therapeutic use of a cosmetic composition for hair growth comprising an effective amount of at least one active agent that activates, enhances, inactivates, blocks or dampens the cellular response of the taste receptor TAS2R4 or interferes with the expression of said receptor is administered to a subject wherein the active agent is selected from
a) any one of the TAS2R4 activating agonists, amarogentin, arborescin, artemorin, azathioprine, chlorpheniramine, denatonium benzoate, diphenidol, dulcoside A, leu-leu-leu, leu-trp, parthenolide, phe-trp, propylthiouracil, quassin, Rebaudioside A, Rebaudioside B, Rebaudioside C, Rubusoside, steviolbioside, Stevioside, sucralose, taurocholic acid, trp-leu, trp-phe, trp-pro, trp-trp, trp-trp-trp, xanthoxin, yohimbine, and (-)epicatechin or a combination thereof, or an aptamer binding to the TAS2R4 receptor and activating or enhancing the receptor to produce a cellular response,or
b) the TAS2R4 inactivating antagonists/inverse agonists carboxymethyllysine (CML), Nα,Nα-bis(carboxymethyl)-l-lysine (BCML), glyoxal-derived lysine dimer (GOLD) and 4-(2,2,3-trimethylcyclopentyl) butanoic acid (GIV3727), or an aptamer binding to the TAS2R4 receptor and inactivating, blocking or dampening the receptor to produce a cellular response or
c) miRNA, siRNA or a ribozyme targeted to the TAS2R4 gene or targeted to the mRNA corresponding to the TAS2R4 gene.

11. Non-therapeutic use according to claim 10, wherein the composition comprises at least one auxiliary agent selected from the group consisting of carriers, recipients, adjuvants, diluents, and disintegrants.

12. Non-therapeutic use according to claims 10 or 11, wherein the auxiliary agent is selected from the group consisting of liposomes, nanoparticles, carboxymethyl cellulose, hydroxyethyl cellulose, mineral oil, petrolatum, glycerin, polysorbat 80, hydroxyethyl starch, dextran, and polyethylene glycol.

13. Non-therapeutic use according to claims 10 to 12, wherein the composition further comprises at least one other active agent being effective in the treatment of hair growth.

14. Non-therapeutic according to one of the claims 10 to 13, wherein the composition is formulated in the form of an ointment, a lotion, a cream, a shampoo, a gel, a solution, a spray, a plaster or a sustained release plaster.

## Patentansprüche

1. Wirkstoff zur Verwendung bei der Behandlung einer Haarwachstumsstörung, wobei der Wirkstoff die zelluläre Antwort des Geschmacksrezeptors TAS2R4 aktiviert, verstärkt, inaktiviert, blockiert oder abschwächt oder mit der Expression des Rezeptors interferiert, und wobei der Wirkstoff ausgewählt ist aus
a) einem der TAS2R4 aktivierenden Agonisten Amarogentin, Arborescin, Artemorin, Azathioprin, Chlorpheniramin, Denatoniumbenzoat, Diphenidol, Dulcosid A, (-)-Epicatechin, Leu-leu-leu, Leu-trp, Parthenolid, phe-trp, Propylthiouracil, Quassin, Rebaudiosid A, Rebaudiosid B, Rebaudiosid C, Rubusosid, Steviolbiosid, Steviosid, Sucralose, Taurocholsäure, trp-leu, trp-phe, trp-pro, trp-trp, trp-trp-trp, Xanthoxin, Yohimbin oder einer Kombination davon, oder einem Aptamer, das an den TAS2R4-Rezeptor bindet und den Rezeptor beim Erzeugen einer zellulären Reaktion aktiviert oder verstärkt, oder
b) einem der TAS2R4 inaktivierenden Antagonisten/inversen Agonisten Carboxymethyllysin (CML), Nα, Nα-Bis(carboxymethyl)-l-lysin (BCML), von Glyoxal abgeleitetes Lysin-Dimer (GOLD) und 4-(2,2,3-trimethylcyclopentyl)butansäure (GIV3727) oder einer Kombination davon oder einem Aptamer, das an den TAS2R4-Rezeptor bindet und den Rezeptor beim Erzeugen einer zellulären Reaktion inaktiviert, blockiert oder abschwächt, oder
c) miRNA, siRNA oder einem Ribozym, das gegen TAS2R4 gerichtet ist.

2. Wirkstoff zur Verwendung bei der Behandlung von Haarwuchs nach Anspruch 1, wobei der Wirkstoff ein Agonist des Geschmacksrezeptors TAS2R4 ist, zur Verwendung bei der Behandlung von unerwünschtem Haarwuchs.

3. Wirkstoff zur Verwendung bei der Behandlung von Haarwuchs nach Anspruch 1, wobei der Wirkstoff ein Antagonist/inverser Agonist des Geschmacksrezeptors TAS2R4 ist, zur Verwendung bei der Behandlung von unerwünschtem Haarausfall.

4. Wirkstoff zur Verwendung bei der Behandlung von Haarwuchs nach einem der Ansprüche 1 bis 3, wobei die zu behandelnde Haarwuchsstörung Effluvium, nicht vernarbende Alopezie, vernarbende Alopezie, Hypertrichose oder Hirsutismus ist.

5. Zusammensetzung zur Verwendung bei der Behandlung von Haarwuchs, wobei die Zusammensetzung mindestens einen Wirkstoff nach einem der Ansprüche 1 bis 3 und mindestens einen Hilfsstoff, ausgewählt aus der Gruppe der Träger, Rezeptoren, Adjuvantien, Verdünnungsmittel und Desintegrationsmittel, enthält.

6. Zusammensetzung zur Verwendung bei der Behandlung von Haarwuchs nach Anspruch 5, wobei das Hilfsmittel ausgewählt ist aus der Gruppe bestehend aus Liposomen, Nanopartikeln, Carboxymethylcellulose, Hydroxyethylcellulose, Mineralöl, Petrolatum, Glycerin, Polysorbat 80, Hydroxyethylstärke, Dextran und Polyethylenglykol.

7. Zusammensetzung zur Verwendung bei der Behandlung von Haarwuchs nach Anspruch 5 oder 6, ferner umfassend mindestens einen weiteren Wirkstoff, der bei der Behandlung von Haarwuchs wirksam ist.

8. Zusammensetzung zur Verwendung bei der Behandlung von Haarwuchs nach einem der Ansprüche 5 bis 7, wobei die Zusammensetzung in Form einer Salbe, einer Lotion, einer Creme, eines Shampoos, eines Gels, einer Lösung, eines Sprays, eines Pflasters oder eines Pflasters mit verzögerter Freisetzung formuliert ist.

9. Nicht-therapeutisches Verfahren zur Regulierung des Haarwachstums, bei dem eine wirksame Menge mindestens eines Wirkstoffs, der die zelluläre Reaktion des Geschmacksrezeptors TAS2R4 aktiviert, verstärkt, inaktiviert, blockiert oder abschwächt oder die Expression dieses Rezeptors stört, an eine Person verabreicht wird, wobei der Wirkstoff ausgewählt ist aus
a) einem der TAS2R4 aktivierenden Agonisten, Amarogentin, Arborescin, Artemorin, Azathioprin, Chlorpheniramin, Denatoniumbenzoat, Diphenidol, Dulcosid A, Leu-leu-leu, Leu-trp, Parthenolid, phe-trp, Propylthiouracil, Quassin, Rebaudiosid A, Rebaudiosid B, Rebaudiosid C, Rubusosid, Steviolbiosid, Steviosid, Sucralose, Taurocholsäure, trp-leu, trp-phe, trp-pro, trp-trp, trp-trp-trp, Xanthoxin, Yohimbin und (-)Epicatechin oder eine Kombination davon, oder ein Aptamer, das an den TAS2R4-Rezeptor bindet und den Rezeptor bei der Erzeugung einer zellulären Reaktion aktiviert oder verstärkt, oder
b) die den TAS2R4-Rezeptor inaktivierenden Antagonisten/umgekehrten Agonisten Carboxymethyllysin (CML), Nα,Nα-Bis(carboxymethyl)-l-lysin (BCML), von Glyoxal abgeleitetes Lysin-Dimer (GOLD) und 4-(2,2, 3-trimethylcyclopentyl)butansäure (GIV3727) oder ein Aptamer, das an den TAS2R4-Rezeptor bindet und den Rezeptor bei der Erzeugung einer zellulären Reaktion inaktiviert, blockiert oder abschwächt oder
c) miRNA, siRNA oder einem Ribozym, das auf das TAS2R4-Gen oder auf die dem TAS2R4-Gen entsprechende mRNA gerichtet ist.

10. Nicht-therapeutische Verwendung einer Zusammensetzung für das Haarwachstum, umfassend eine wirksame Menge mindestens eines Wirkstoffs, der die zelluläre Reaktion des Geschmacksrezeptors TAS2R4 aktiviert, verstärkt, inaktiviert, blockiert oder abschwächt oder die Expression des Rezeptors stört, zur Verabreichung an eine Person, wobei der Wirkstoff ausgewählt ist aus
a) einem der TAS2R4 aktivierenden Agonisten, Amarogentin, Arborescin, Artemorin, Azathioprin, Chlorpheniramin, Denatoniumbenzoat, Diphenidol, Dulcosid A, Leu-leu-leu, Leu-trp, Parthenolid, phe-trp, Propylthiouracil, Quassin, Rebaudiosid A, Rebaudiosid B, Rebaudiosid C, Rubusosid, Steviolbiosid, Steviosid, Sucralose, Taurocholsäure, trp-leu, trp-phe, trp-pro, trp-trp, trp-trp-trp, Xanthoxin, Yohimbin und (-)Epicatechin oder eine Kombination davon, oder einem Aptamer, das an den TAS2R4-Rezeptor bindet und den Rezeptor bei der Erzeugung einer zellulären Reaktion aktiviert oder verstärkt, oder
b) die den TAS2R4-Rezeptor inaktivierenden Antagonisten/umgekehrten Agonisten Carboxymethyllysin (CML), Nα,Nα-Bis(carboxymethyl)-l-lysin (BCML), von Glyoxal abgeleitetes Lysin-Dimer (GOLD) und 4-(2,2, 3-trimethylcyclopentyl)butansäure (GIV3727) oder ein Aptamer, das an den TAS2R4-Rezeptor bindet und den Rezeptor bei der Erzeugung einer zellulären Reaktion inaktiviert, blockiert oder abschwächt oder
c) miRNA, siRNA oder einem Ribozym, das auf das TAS2R4-Gen oder auf die dem TAS2R4-Gen entsprechende mRNA gerichtet ist.

11. Nicht-therapeutische Verwendung nach Anspruch 10, wobei die Zusammensetzung mindestens ein Hilfsmittel umfasst, das aus der Gruppe ausgewählt ist, die aus Trägern, Rezeptoren, Adjuvantien, Verdünnungsmitteln und Sprengmitteln besteht.

12. Nicht-therapeutische Verwendung nach Anspruch 10 oder 11, wobei das Hilfsmittel ausgewählt ist aus der Gruppe bestehend aus Liposomen, Nanopartikeln, Carboxymethylcellulose, Hydroxyethylcellulose, Mineralöl, Petrolatum, Glycerin, Polysorbat 80, Hydroxyethylstärke, Dextran und Polyethylenglykol.

13. Nicht-therapeutische Verwendung nach einem der Ansprüche 10 bis 12, wobei die Zusammensetzung weiterhin mindestens einen anderen Wirkstoff enthält, der bei der Behandlung von Haarwachstum wirksam ist.

14. Nicht-therapeutische Verwendung nach einem der Ansprüche 10 bis 13, wobei die Zusammensetzung in Form einer Salbe, einer Lotion, einer Creme, eines Shampoos, eines Gels, einer Lösung, eines Sprays, eines Pflasters oder eines Pflasters mit verzögerter Freisetzung formuliert ist.

## Revendications

1. Agent actif destiné à être utilisé dans le traitement d'un trouble de la croissance pilaire, où ledit agent actif active, renforce, inactive, bloque ou atténue la réponse cellulaire du récepteur gustatif TAS2R4 ou interfère avec l'expression dudit récepteur, et où l'agent actif est choisi parmi
a) l'un quelconque des agonistes activateurs de TAS2R4 que sont l'amarogentine, l'arborescine, l'artémorine, l'azathioprine, la chlorphéniramine, le benzoate de dénatonium, le diphénidol, le dulcoside A, l'(-)-épicatéchine, la leu-leu-leu, le leu-trp, le parthénolide, le phe-trp, le propylthiouracile, la quassine, le rébaudioside A, le rébaudioside B, le rébaudioside C, le rubusoside, le stéviolbioside, le stévioside, le sucralose, l'acide taurocholique, la trp-leu, la trp-phe, la trp-pro, le trp-trp, le trp-trp-trp, la xanthoxine, l'yohimbine, ou une combinaison quelconque de ceux-ci, ou un aptamère se liant au récepteur TAS2R4 et activant ou renforçant le récepteur pour produire une réponse cellulaire, ou
b) l'un quelconque des antagonistes/agonistes inverses inactivateurs de TAS2R4 que sont la carboxyméthyl-lysine (CML), la Nα,Nα-bis(carboxyméthyl)-L-lysine (BCML), le dimère de lysine dérivé du glyoxal (GOLD) et l'acide 4-(2,2,3-triméthylcyclopentyl)butanoïque (GIV3727) ou une combinaison quelconque de ceux-ci, ou un aptamère se liant au récepteur TAS2R4 et inactivant, bloquant ou atténuant le récepteur pour produire une réponse cellulaire, ou
c) un miARN, un siARN ou un ribozyme ciblé sur TAS2R4.

2. Agent actif destiné à être utilisé dans le traitement de la croissance pilaire selon la revendication 1, où l'agent actif est un agoniste du récepteur gustatif TAS2R4 destiné à être utilisé dans le traitement d'une croissance des poils indésirables.

3. Agent actif destiné à être utilisé dans le traitement de la croissance pilaire selon la revendication 1, où l'agent actif est un antagoniste/agoniste inverse du récepteur gustatif TAS2R4, destiné à être utilisé dans le traitement d'une perte de cheveux indésirable.

4. Agent actif destiné à être utilisé dans le traitement de la croissance pilaire selon l'une des revendications 1 à 3, où le trouble de la croissance pilaire à traiter est l'effluvium, l'alopécie non cicatricielle, l'alopécie cicatricielle, l'hypertrichose ou hirsutisme.

5. Composition destinée à être utilisée dans le traitement de la croissance pilaire, ladite composition comprenant au moins un agent actif selon l'une des revendications 1 à 3 et au moins un agent auxiliaire choisi dans le groupe constitué par les véhicules, récipients, adjuvants, diluants et désintégrants.

6. Composition destinée à être utilisée dans le traitement de la croissance pilaire selon la revendication 5, dans laquelle l'agent auxiliaire est choisi dans le groupe constitué par les liposomes, les nanoparticules, la carboxyméthylcellulose, l'hydroxyéthylcellulose, l'huile minérale, la vaseline, la glycérine, le polysorbate 80, l'hydroxyéthylamidon, le dextrane et le polyéthylèneglycol.

7. Composition destinée à être utilisée dans le traitement de la croissance pilaire selon la revendication 5 ou 6, comprenant en outre au moins un autre agent actif qui est efficace dans le traitement de la croissance pilaire.

8. Composition destinée à être utilisée dans le traitement de la croissance pilaire selon l'une des revendications 5 à 7, ladite composition étant formulée sous forme d'une pommade, d'une lotion, d'une crème, d'un shampoing, d'un gel, d'une solution, d'une pulvérisation, d'un emplâtre ou d'un emplâtre à libération prolongée.

9. Procédé non thérapeutique de régulation de la croissance pilaire, dans lequel une quantité efficace d'au moins un agent actif qui active, renforce, inactive, bloque ou atténue la réponse cellulaire du récepteur gustatif TAS2R4 ou qui interfère avec l'expression dudit récepteur est administrée à un sujet, dans lequel l'agent actif est choisi parmi
a) l'un quelconque des agonistes activateurs de TAS2R4 que sont l'amarogentine, l'arborescine, l'artémorine, l'azathioprine, la chlorphéniramine, le benzoate de dénatonium, le diphénidol, le dulcoside A, la leu-leu-leu, le leu-trp, le parthénolide, le phe-trp, le propylthiouracile, la quassine, le Rébaudioside A, le Rébaudioside B, le Rébaudioside C, le Rubusoside, le Stéviolbioside, le Stévioside, le sucralose, l'acide taurocholique, la trp-leu, la trp-phe, la trp-pro, le trp-trp, le trp-trp-trp, la xanthoxine, l'yohimbine et l'(-)-épicatéchine, ou une combinaison quelconque de ceux-ci, ou un aptamère se liant au récepteur TAS2R4 et activant ou renforçant le récepteur pour produire une réponse cellulaire, ou
b) les antagonistes/agonistes inverses inactivateurs de TAS2R4 que sont la carboxyméthyl-lysine (CML), la Nα,Nα-bis(carboxyméthyl)-L-lysine (BCML), le dimère de lysine dérivé du glyoxal (GOLD) et l'acide 4-(2,2,3-triméthylcyclopentyl)butanoïque (GIV3727), ou un aptamère se liant au récepteur TAS2R4 et inactivant, bloquant ou atténuant le récepteur pour produire une réponse cellulaire, ou
c) un miARN, un siARN ou un ribozyme ciblé sur le gène de TAS2R4 ou ciblé sur l'ARNm correspondant au gène de TAS2R4.

10. Utilisation non thérapeutique d'une composition cosmétique destinée à la croissance pilaire, comprenant une quantité efficace d'au moins un agent actif qui active, renforce, inactive, bloque ou atténue la réponse cellulaire du récepteur gustatif TAS2R4 ou qui interfère avec l'expression dudit récepteur, administrée à un sujet, dans lequel l'agent actif est choisi parmi
a) l'un quelconque des agonistes activateurs de TAS2R4 que sont l'amarogentine, l'arborescine, l'artémorine, l'azathioprine, la chlorphéniramine, le benzoate de dénatonium, le diphénidol, le dulcoside A, la leu-leu-leu, le leu-trp, le parthénolide, le phe-trp, le propylthiouracile, la quassine, le Rébaudioside A, le Rébaudioside B, le Rébaudioside C, le Rubusoside, le stéviolbioside, le Stévioside, le sucralose, l'acide taurocholique, la trp-leu, la trp-phe, la trp-pro, le trp-trp, le trp-trp-trp, la xanthoxine, l'yohimbine et l'(-)-épicatéchine, ou une combinaison quelconque de ceux-ci, ou un aptamère se liant au récepteur TAS2R4 et activant ou renforçant le récepteur pour produire une réponse cellulaire, ou
b) les antagonistes/agonistes inverses inactivateurs de TAS2R4 que sont la carboxyméthyl-lysine (CML), la Nα,Nα-bis(carboxyméthyl)-L-lysine (BCML), le dimère de lysine dérivé du glyoxal (GOLD) et l'acide 4-(2,2,3-triméthylcyclopentyl)butanoïque (GIV3727), ou un aptamère se liant au récepteur TAS2R4 et inactivant, bloquant ou atténuant le récepteur pour produire une réponse cellulaire, ou
c) un miARN, un siARN ou un ribozyme ciblé sur le gène de TAS2R4 ou ciblé sur l'ARNm correspondant au gène de TAS2R4.

11. Utilisation non thérapeutique selon la revendication 10, dans laquelle la composition comprend au moins un agent auxiliaire choisi dans le groupe constitué par les véhicules, récipients, adjuvants, diluants et désintégrants.

12. Utilisation non thérapeutique selon la revendication 10 ou 11, dans laquelle l'agent auxiliaire est choisi dans le groupe constitué par les liposomes, les nanoparticules, la carboxyméthylcellulose, l'hydroxyéthylcellulose, l'huile minérale, la vaseline, la glycérine, le polysorbate 80, l'hydroxyéthylamidon, le dextrane et le polyéthylèneglycol.

13. Utilisation non thérapeutique selon les revendications 10 à 12, dans laquelle la composition comprend en outre au moins un autre agent actif qui est efficace dans le traitement de la croissance pilaire.

14. Utilisation non thérapeutique selon l'une des revendications 10 à 13, dans laquelle la composition est formulée sous forme d'une pommade, d'une lotion, d'une crème, d'un shampoing, d'un gel, d'une solution, d'une pulvérisation, d'un emplâtre ou d'un emplâtre à libération prolongée.
